# EUROPEAN PATENT SPECIFICATION

(11) **EP 1 877 201 B1**
(45) Date of publication and mention of the grant of the patent: **20.06.2012**
(21) Application number: 05745350.8
(22) Date of filing: 03.05.2005
(51) Int. Cl.: C12P 5/02, B09B 3/00

(54) **Stimulation of methane production by an isolated anaerobic microbial consortium**
Stimulation der Methanproduktion durch ein isoliertes anaerobes mikrobielles Konsortium
Stimulation de la production de méthane par un consortium microbien anaérobie isolé

(43) Date of publication of application: 16.01.2008
(73) Proprietor: Luca Technologies Inc., Golden, CO 80401 (US)
(72) Inventor: PFEIFFLER, Robert S., Parker, CO 80108 (US); ULRICH, Glenn, Golden, CO 80403 (US); VANZIN, Gary, Arvada, CO 80005 (US); DANNAR, Verlin, Sheridan, WO 82801 (US); DEBRUYN, Roland,P, Highlands Ranch, CO 80126 (US); SZALOCZI, Eric,L., Denver, CO 80204 (US)
(74) Representative: Woolley, Lindsey Claire
(86) International application number: PCT/US2005/015188
(87) International publication number: WO 2006/118569

(56) References cited:
- US-A- 5 490 634
- US-A1- 2002 102 673
- US-A1- 2004 033 557
- US-B2- 6 543 535
- ULRICH, G.A. ET AL.: "Active Biogenisis: Microbes are making natural gas in Wyoming's Powder River Basin" ENERGY MAGAZINE, vol. 30, no. 2, April 2005 (2005-04), pages 21-26, XP008128250

## Description

### BACKGROUND OF THE INVENTION

It is well-known that methane can be produced in carbonaceous formations by microbial metabolism. [Jones, W. J. et al. (1987) Microbiol. Rev. 51, 135]. Many species of methanogens, the microorganisms (MO's) that have the capability to convert a variety of substrates including CO₂, acetate, H₂, methanol, methylamine or methyl thiols to methane, are now known [Ferry, J. G. et al. (1992) Crit. Rev. in Biochem. and Mol. Biol. 27:473-503]. Methanogens are obligate anaerobes [Ferry, J. G., in Biochemistry and Physiology of Anaerobic Bacteria Lars, G. Ljungdahl et al., eds. 143-156, Springer-Verlag, New York (2003)]. Even brief exposures to low concentrations of oxygen greatly reduces their viability [Tanner, R.S. et al., (1983) Appl Microbiol. 4:305-312]. Methodologies for preventing oxygen exposure to methanogenic consortia in laboratory settings have been established [Kiener, A. et al., in Manual of Environmental Microbiology C.J. Hurst et al., eds. 52-71, ASM Press, Washington, D.C. (1997)] but the same is not true for harvesting, handling, and transporting methanogenic consortia in the field.

A complete process of methanogenesis requires metabolic capabilities of other MO's, besides methanogens, in order to convert the higher molecular weight aliphatic and aromatic hydrocarbons of a carbonaceous formation into CO₂, acetate, H₂, methanol, methylamine, methylthiols or other suitable substrate for a methanogen. The full complement of MO's whose combined metabolism in the formation yields methane is termed a methanogenic consortium. Other than methanogens, the MO's of a methanogenic consortium are poorly characterized and are thought to differ depending on the carbonaceous composition and the environment of the formation.

Measurable methanogenesis supported by carbonaceous materials has been observed under laboratory conditions. Galand et al. [Galand, P. E. et al. (2003) Environmental Microbiol. 5:1133-1143] measured CH₄ output from samples of peat obtained from a fen, Isolation of MO's from the sample was not reported. Volkwein [U. S. Patent 5,424,195, issued June 13, 1995] reported methanogenesis from abandoned coal mine sediment supplemented with a nutrient medium. MO's of the sediment were not separated from the sediment. See also Ulrich G.A. et al, Energy Magazine vol 30, no. 2, April 2005, pages 21-26.

The general concept of enhancing production of biogenic methane from a carbonaceous formation has been suggested previously [Raabe, S., Denver Post, Nov.17, 2004, p.1C]. Volkwein, *supra*, reported isolating a methanogenic sediment from an abandoned coal mine into which sewage had been discharged for an unspecified time period. For mine cavities having a particular history, from a time where a nutrient source was present to a time where the nutrient source was absent, sediment could be collected which was alleged to be methanogenic in the presence of bituminous coal. No supporting data were disclosed. Scott, et al. Pub. No. US 2004/0033557 A1 (Feb.19, 2004) generally describes introducing subsurface fractures in a deposit of coal, carbonaceous shale or organic-rich shale and injecting various modifications including a consortium of selected anaerobic biological microorganisms, nutrients, carbon dioxide and other substrates for *in situ* conversion of organic compounds in said formation into methane and other compounds. The disclosure does not specifically teach how to obtain "selected" bacterial consortia; however, the reference suggests that collection of bacteria from formation waters may result in collection of only a few species rather than a representative sample of bacterial consortia. No supporting data of methane generation were reported. Menger, et al. U.S. Patent 4,845,034 described carrying out a biochemical reaction in a subterranean cavity formed in a salt formation, limestone cavity or other earthen rock or sandstone formation. A feedstock of finely-divided, hot-alkali-treated coal would be inoculated under controlled conditions with a culture of microorganisms including acid formers and methanogens to produce methane. No data reporting methane biosynthesis were reported.

Many studies report isolating and characterizing MO's in naturally-occurring waters including ground water. Pickup et al. [Pickup, R. W. et al. (2001) J. Contam. Hydrol. 53:269-284] reported a detailed study of MO's in an aquifer polluted by a plume of phenolic material emanated from a single known source. Water from the aquifer was sampled at several depths from two boreholes within the plume. Details of the sampling method were disclosed by Thornton et al. [Thornton, S. F. et al. (2001) J. Contam. Hydrol. 53:233-267]. Water samples were filtered though 0.22 µm polycarbonate filters, assayed for total MO count by acridine orange staining and by counting colonies of culturable MO's. The number of culturable MO's was 1% or less of the total measured by acridine orange staining. The authors used a variety of techniques to assess numbers and activities of various MO classes and to evaluate difference that varied with sample depth and phenolic concentration. The presence of methanogens was revealed using polymerase chain reaction analyses to amplify known methanogen-specific sequences. Methane generation was not reported.

Various filtration techniques have been reported for collecting MO's from groundwater. Schulz-Makuch et al [Schulze-Makuch, D. et al. (2003) Ground Water Monitor. and Remed. 23:68-75] compared the efficacy of filter packs containing surfactant-modified zeolite or iron oxide-coated sand for removing *E. coli* and MS-2 virus from contamined groundwater. The surfactant modified zeolite removed both the bacteria and the virus, but the iron oxide-coated sand was ineffective. Lillis et al [Lillis, T. O. et al. (2001) Lett. Appl. Microbiol. 32:268-272] compared membrane filters to collect MO's from groundwater. Recovery was measured by comparing colony counts of MO's cultured after filtration. Filters of pore size 0.45 µm recovered about 90% of the MO's; however, the remaining MO's were recovered only after filtration through 0.22 µm filters. Filtration can remove both viable and non-viable cells. Culture conditions may not be suitable for growing many, or even most of the filterable MO's. Kunicka-Goldfinger et al. (1977) Acta Microbiol. Polonica 26:199-205, reported that an agar plate method of counting colony forming units (cfu) accounted for only 20-25% of organisms counted by direct staining of MO's isolated by filtration from lake waters. A "semi-continuous" method of culturing cells on the filters, wherein the cells were periodically exposed to filtered lake water to re-supply natural nutrients and remove waste products yielded significantly higher numbers of culturable microorganisms.

Tangential filtration has been reported for isolation of proteins and microorganisms. U.S. Patent Application 10/703,150, published June 24, 2004 disclosed concentrating a suspension of microalgae by passing the suspension through a tangential filtering device. EPA document 815-0-03-008, June 2003 provides extensive technical and performance data for membrane filtration, including tangential flow filtration, in water purification systems.

To date, most contributions to the art have emphasized nutritional amendments *in situ,* or culturing microorganisms prior to injection into a formation or introducing fractures in a formation. Techniques for isolating a methanogenic consortium and demonstrating methanogenesis from an isolated consortium remain as problems inadequately addressed in the prior art.

### SUMMARY OF THE INVENTION

The invention is a method for stimulating biogenic methane production from a carbonaceous substrate. Methane production according to the invention can be stimulated *in situ* in an underground formation of carbonaceous material, or in extracted carbonaceous material. By contacting the carbonaceous material with a methanogenic consortium prepared according to the invention methane synthesis has been demonstrated to occur, even in the case of carbonaceous material which was formerly deemed unproductive. The method does not depend on providing exogenous amendments, multiplying consortium MO's in culture, addition of substrates, or upon prior nutrient injection, structural modification of the formation, or prior chemical modification of the carbonaceous material, although such steps are not excluded.

The invention also includes a method of preparing a concentrate of micro-organisms ("MO's" hereinafter) comprising a consortium of MO's, which method comprises the steps of, a) extracting anaerobic formation water containing said MO's from an underground carbonaceous formation, b) providing liquid transport means for transporting said water while maintaining an anaerobic state, c) providing collection means for collecting said MO's in an anaerobic state from said water, d) transporting said water through said collection means, whereby MO's are collected from said water, and collecting said MO's in a concentrated form in said water from the collection means, whereby a concentrate of said MO's is prepared. MO's prepared according to the invention are essentially sediment-free.

Data presented herein demonstrate that anaerobic formation water obtained from any of several underground carbonaceous formations carries numerous MO's including a variety of MO species. Further, the data demonstrate that included among the MO's that can be collected from such water are all the species that make up a consortium capable of using a carbonaceous substrate, including coal, as a substrate to produce methane, under anaerobic conditions. Even though the number of MO's per liter of formation water may be low, large amounts of MO's can be accumulated by processing large volumes of water to collect the MO's therefrom.

Micro-organisms concentrated according to the invention are useful for biogenic production of methane from a carbonaceous substrate. Concentrated MO's can be used to generate methane under controlled conditions in an above-ground bioreactor, or used to enhance *in situ* methanogenesis. In either case, the methane that is generated can be used as a feedstock for chemical synthesis or used directly as a clean burning fuel. The biogenic production of methane by the present invention requires minimal input of external energy.

### BRIEF DESCRIPTION OF THE FIGURES

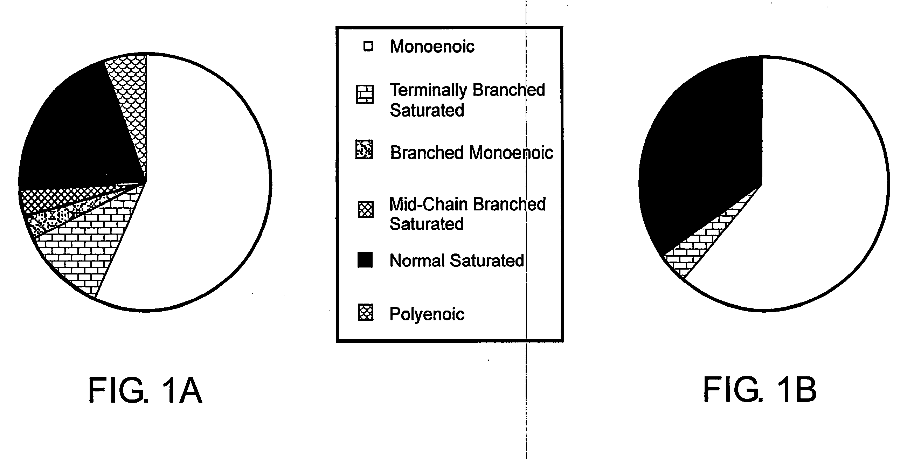

Fig. 1 shows two pie graphs showing the relative abundance of different microbial phospholipid types by conventional phospholipid fatty acid analysis (PLFA), comparing cells isolated from waters produced from The Tongue River Member of the Fort Union Formation located in the western edge of the Power River Basin of NE Wyoming, (left hand graph) with cells present in coals of the same formation (right hand graph). Total microbial biomass was 1.2 x 10⁵ cells/ml Tongue River water, and 3 x 10⁶ cells/g Tongue River coal, respectively.

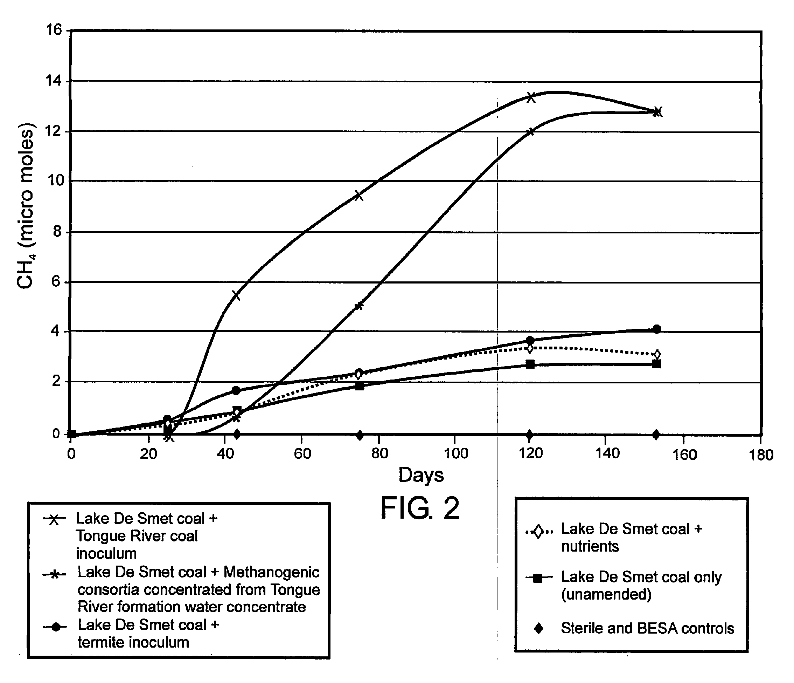

Fig. 2 is a graph of methane production as a function of time, from Lake De Smet coal, as described in Example 1, under various conditions as shown.

### DETAILED DESCRIPTION OF THE INVENTION

The term "micro-organism" is used in its broadest and generally understood sense to mean any living organism that is too small to be seen with the naked eye. MO's are found in almost all samples of underground material, either dispersed in subterranean water or adsorbed on or adhering to solid material. Of particular interest in the present invention are MO's suspended in underground water, namely water obtained from carbonaceous formations, namely, water obtained from an anaerobic region of a methane-producing carbonaceous formation. Such water is termed "anaerobic formation water" herein.

A "carbonaceous formation" is any place on or under the earth's surface whose composition is rich in hydrocarbons, including but not limited to coal, bitumen, oil shale, carbonaceous shale, tar sands, peat, oil and/or gas deposits, sediments rich in organic matter and the like. "Formation water" is water found within, or obtained from a carbonaceous formation. "Anaerobic" formation water is characterized as having little or no dissolved oxygen, in general no more than 4 mg/L, preferably less than 2 mg/L, most preferably less than 0.1 mg/L, as measured at 20 degrees C and 760 mmHg barometric pressure. During application of the present invention, higher levels of dissolved oxygen, greater than 4 mg/L, can be tolerated without appreciably degrading consortium performance, for limited times or in certain locations such as a surface layer in a storage or settling tank. Dissolved oxygen can be measured by well-known methods, such as by commercially-available electrodes, or by the well-known Winkler reaction. It will be understood that carbonaceous formations can have both aerobic regions and anaerobic regions. For example, a coal deposit is likely to be aerobic near a surface exposed to air by mining activity. At a depth below the exposed surface, the deposit, including associated formation waters becomes anaerobic. Such water is what is termed herein "anaerobic formation water."

Methane is generated in a carbonaceous formation by metabolic activity of MO's within the formation. Typically, a plurality of MO species act to degrade complex hydrocarbons in the formation to metabolic precursors of methane such as to acetate and/or CO₂ which can be further metabolized to methane by various species of methanogens. The MO's whose concerted metabolic actions result in hydrocarbon degradation and methane production are termed a "methanogenic consortium" herein. Active MO's of a methanogenic consortium are likely to be most abundant in formation water of a methane-producing formation.

"Contacting" refers to any process which results in bringing a methanogenic consortium into surface contact with carbonaceous material.

"Liquid transport means" includes any device or combination of devices for transporting anaerobic formation water from one location to another. Pumping devices for withdrawing water from the formation and pressure devices for expelling water from the formation are contemplated. Pumping means can be operated mechanically, hydraulically, pneumatically or by fluid expansion. Pumping means include without limitation:

Any dynamic pumping device, as described below:
- centrifugal pumps, including axial flow [single-stage or multi-stage; closed impeller; open impeller (either fixed-pitch or variable-pitch); including any combination of these characteristics]; mixed flow and/or radial flow [single suction or double suction; self-priming, non-priming, single-stage, or multi-stage; open-impeller, semi-open impeller, or closed-impeller; including any combination of these characteristics]; and peripheral [single-stage or multi-stage; self-[priming or non-priming; including any combination of these characteristics];
- jet pumps;
- gas lift pumps;
- hydraulic ram pumps;
- electromagnetic pumps.

Any reciprocating displacement pumping device, as described below:
- piston or plunger pumps, including steam [simplex, duplex, triplex or multiplex]; and power [single-acting or double-acting; simplex, duplex, triplex or multiplex; including any combination of these characteristics];
- pumps utilizing check valves (whether fixed, mobile, or any combination of these characteristics) including hinged barriers, mobile balls or mobile pistons of appropriate shape, with associated containment devices;
- diaphragm pumps, including simplex, duplex and multiplex, fluid-operated and mechanically-operated, and including any combination of these characteristics.

Any rotary displacement pumping device, as described below:
- pumps equipped with a single rotor, including vane, piston, flexible member, screw and peristaltic;
- pumps equipped with multiple rotors, including gear, lobe, circumferential piston, and screw.

The anaerobic state of formation water can be maintained during pumping, filtration and water storage by maintaining a closed system throughout and by injecting an inert gas such as argon, nitrogen or helium into the system to minimize contamination by oxygen. Preferred pumping means are low pressure pumps such as vein, fin or rotary pumps that use needle, ball or butterfly valves. Typically, pumps used for removing water from coal deposits are submersible. As is understood in the art, water pumped from a deep well releases dissolved methane and other gases as it is brought to the surface. Therefore, the use of submersible pumps combined with gas collection devices at the well-head are preferred by those skilled in the art. Preferred materials for transport and storage of anaerobic water are those which are oxygen impermeable and chemically inert, to minimize diffusion into the water of oxygen or other materials that could affect growth, viability or metabolic functions of MO's in the water. Examples of preferred materials include butyl rubber, viton, glass, steel and stainless steel. Examples of non-preferred materials include oxygen permeable tubing including nylon, tygon, silicone and polyvinyl chloride. The inventors have found that it is not possible to maintain anoxic conditions with these oxygen permeable materials.

It will be understood in the art that the choice of an optimal pumping device for a given formation will depend upon the volume of water to be processed, the depth of the anaerobic formation water source, temperature of the water, mineral content of the water, rate that water flow in the formation replaces the water removed, and the composition of the carbonaceous formation.

In general, pumps should be chosen that do not introduce air and do not create significant forces that could shear MO's. All such features will be understood by those skilled in the art, taking into account the need to avoid oxygen intrusion into the water being pumped, and to maintain viability of MO's that exist in the water and therefore to avoid introducing physical or chemical factors that would compromise viability. It will be understood that pumping can occur in stages, using different pumping means for different stages. Monitoring devices can be used to assist in coordinating pumping rates and filtration rates, to regulate inert gas input as needed, measure oxygen concentration, cell concentration and transmembrane pressure, and to measure water levels and head space volumes in any storage or holding tanks.

Microorganisms can be collected from aqueous fluids by a variety of means, including filtration, centrifugation, flocculation, affinity binding, and the like. Preferred collection means are filtration and centrifugation, since these do not depend on specific chemical or biochemical properties of the MO's in the fluid and they can be adapted to continuous flow for processing large volumes of fluid. Filtration is especially preferred because it is more readily adaptable to maintaining an anaerobic state. Filtration is the process of collecting, fractionating, or concentrating particles, molecules, or ions within or from a fluid by forcing the fluid material through a porous or semi-porous barrier. The force can be pressure differential, vacuum, concentration gradient, valence or electrochemical affinity. The fluid can be either liquid or gas. Two common types of filtration include:
a. Dead-end (linear) filtration; and
b. Tangential (cross) flow filtration.

Filters are generally of two types, either depth filters or membrane filters. Depth filters do not have absolute pore sizes, but trap some particles on the surface of the filter, some particles by random entrapment and adsorption within the filter, and possible particle retention through charge. Membrane filters generally function by retaining particles through an absolute pore size. Most MO's can be retained on filters having a maximum pore size of about 0.2 microns.

Factors that affect filtration rate include pore size, filter composition, density of MO's in the water and presence of contaminating particles. Particles other than MO's that are too large to pass through the pores of a filter can clog the filter, particularly if present in excess of the MO's themselves. Particles larger than MO's can be removed prior to collecting the MO's by means known in the art, often by a pre-filtration process that does not significantly remove MO's from the water. Settling tanks can also be used for removing large particles. Filters having a maximum pore size less than 0.2 microns have reduced flow rate and are less preferred.

Filter materials will be chosen to provide optimum pore size and hydrophobicity. While MO's are more easily removed from hydrophobic filter materials, the flow rate of water through a filter membrane is lower for highly hydrophobic membranes. Flow rate is also reduced as pore size is reduced. Filters having nominal pore size of 0.45 microns will retain most MO's. However, a significant number of small MO's have been reported in ground-water samples [see, e.g. Lillis, et al, *supra*]. Preferred filters will have pore sizes of about 0.2 - 0.45 microns and preferably be composed of polyether sulfones, polysulfones, cellulose acetate or PVDF. Filters composed of nylon or nitrocellulose are not preferred.

Efficient processing of large water volumes can be achieved by increasing filter area. The optimum filter area to be employed will depend on the volume to be processed and the desired rate of processing. Various configurations for maximizing filter area within a given sized device are known in the art. A common configuration is a pleated sheet rolled into a cylinder. Another common configuration is a flat sheet rolled into a spiral around a cylindrical core. Other configurations are known in the art and can be adapted for use in the present invention by those skilled in the art. Tangential, or cross-flow filtration is an effective expedient for filtering large volumes of water. Suitable filters available commercially having the described characteristics are polyether sulfone hollow fiber filters having 0.2 microns nominal pore size. For laboratory scale filtrations, filters having 2000 cm² surface area, such as Spectrum Technologies part no. M22M-301-01 N have been successfully employed. For large scale processing, modules of Spectrum Technologies M22M-600-01 N with a 5200 cm² surface area can be linked in parallel. The foregoing are provided as non-limiting examples. Other filter types and filter materials are known to those of skill in the art.

During tangential flow filtration, water is removed from fluids that are recirculated through a tangential flow filter. This yields water (often termed retentate) that is concentrated with microorganisms. The retentate can be pumped into a storage tank that has been sparged with an inert gas, after which the tangential flow filtration of additional water can resume. Microorganisms can also be removed from filter means by washing or backwashing, to provide the MO's at higher concentration than in the formation water. If desired, further concentration can be achieved by additional rounds of filtration. In addition, MO's obtained in a semi-concentrated form by filtration can be further concentrated by centrifugation or dehydration to yield a paste or slurry of packed or highly concentrated cells. By application of such processes, formation water having relatively low biomass, as little as 10² cell/ml can be processed to yield MO's at concentrations of 10⁸/ml or more. Transporting anaerobic formation water through such separation means, whether in single or plural stages, whether by use of one or more of such means in tandem, all while maintaining an anaerobic state, can yield viable, active methanogenic consortia present in the formation water in a concentrated form. Anaerobic formation water from which viable methanogenic consortia were isolated was clear to the naked eye. Nevertheless, it will be understood that small particles of organic or inorganic matter may be retained along with MO's in the course of filtration and centrifugation carried out as described herein. Microscopic analysis of MO concentrates prepared according to the invention revealed numerous discrete rod-shaped cells and few if any irregular particles or particles with cells attached. The term "essentially sediment-free" is used to denote a preparation of cells that contains at least 50% discrete or clustered cells, unattached to debris or non-cellular particles. Cell viability can be evaluated by methods known in the art, for example by assessing all membrane integrity based on permeability to a cell permeant DNA-binding fluorescent dye.

The inventors herein have made the surprising discovery that all the MO species that make up a methanogenic consortium capable of generating methane from the carbonaceous matter in their formation of origin, can be recovered in a viable, active state from formation water, under anaerobic conditions. Formation water is abundant. The present disclosure provides abundant consortium MO's by simple, inexpensive means, and without the use of expensive fermentation processes. The consortia MO's in concentrated form, are useful for inoculating carbonaceous materials, *in situ* or above ground, to generate methane or to amplify the generation of methane using the carbonaceous materials as substrate. The consortia have been demonstrated herein to stimulate methanogenesis from essentially non-productive coal, without prior analysis of individual strains or their nutritional requirements, without added nutrients and without growing the cells in culture prior to contacting the coal with consortium MO's.

The practice of the invention is further described in the following Example.

### Example 1

### Quantification and characterization of microbial biomass in formation waters of the Powder River Basin (PRB) coal bed methane development area.

Four coal bed methane (CBM) water samples and six coal samples collected from the Tongue River area of the Powder River Basin in Wyoming were analyzed for phospholipid fatty acid analysis (PLFA). PLFA has proven to be an accurate tool for quantifying viable microbial biomass. The formation water samples were placed into sterile 4L bottles with no headspace and spiked with a sterile sodium sulfide solution (to 0.5 mM) to preserve anoxic conditions. Oxygen exposure of the coal samples was minimized by storing them in gas tight cylinders that were purged with argon. Figure 1 illustrates the microbial biomass estimates and community structure in both formation water and coal samples based on PLFA abundance and makeup respectively.

Microbial biomass in the formation water samples ranged from 7.65 x 10⁴ cells/ml to 1.69 x 10⁵ cells/ml with an average of 1 x 10⁵ cells/ml. For comparison, microbial biomass in the coal samples ranged from 1.4 x 10⁶ cells/g coal to 9.5 × 10⁶ cells/g coal with an average of 3 x 10⁶ cells/g coal. The microbial communities in formation water and in coal samples had similar PLFA compositions. These data indicate that the primary groups of microorganisms in the tested coals are also present in the associated formation waters.

### Evaluation of methods for concentrating microorganisms from formation waters of the PRB.

Microorganisms were concentrated from formation water obtained from a coal seam within the Powder River Basin (Tongue River area) using a hollow fiber tangential flow filtration column, and for comparison, by centrifugation.

The centrifugation procedure included several cycles of centrifuging formation water at 5500 X G for 10 minutes in 60 ml centrifuge tubes. All sample manipulations were conducted in an anaerobic glove bag. A total volume of 1330 ml of formation water was centrifuged to 18.4 ml. The sample was analyzed by microscopy to ensure that microorganisms represented most of the turbidity (vs. coal fines or other particles).

The tangential flow procedure consisted of re-circulating 4 L Tongue River water through a tangential flow filtration column (0.2 µm pore size; 520 cm² surface area, by Spectrum Labs, part # M22E=101-015). Viton tubing, which has a very low oxygen permeability, and air tight (Swagelock) fittings were used for the flow lines and fittings respectively. A glass carboy purged with argon was used as the formation water holding vessel.

Three tests were performed to concentrate MO's from formation water at varying tangential total flow rates ranging from 370 ml/min to 2,300 ml/min (see table 1). The permeate (filtered water leaving the tangential filter) flow rate ranged from 200 ml/minute to 614 ml/min. The efficiency of tangential flow for concentrating microorganisms from the formation water relative to centrifugation ranged from 95% to 155% (see the final column of table 1). The final microbial biomass in the cell concentrates is in agreement with the cell concentration in the formation water. Thus, both centrifugation and tangential flow filtration were effective at concentrating microbial biomass from the formation water. Of particular interest is the final tangential flow test during which over 2 liters of water was filtered within approximately 3 minutes using a very small tangential flow filtration column. Concentrated MO's were active in formation water under refrigeration at 37°F for at least 1 week.

**Table 1. Comparison of centrifugation and tangential flow filtration for concentrating microbial biomass from formation water collected from a coal bed methane well.**

| **Total Flow** | **Permeate Flow** | | **Cells/ml** | **cell recovery efficiency relative to centrifugation** |
|---|---|---|---|---|
| | | **Volumetric** | | |
| **Rate ml/min** | **Rate (ml/min)** | **Concentration** | **in concentrate** | |
| centrifuged | centrifuged | 80x | 5.8 x 10⁸ | NA |
| 370 | 200 ml/min | 133x | 9.2 x 10⁶ | 95% |
| 400 | 168 ml/min | 82x | 6.2 x 10⁶ | 108% |
| 2,300 | 614 ml/min | 82x | 9.0 x 10⁶ | 155% |

### Methanogenesis stimulated by microorganisms concentrated from CBM waters.

Experiments were conducted to test whether the introduction of microorganisms concentrated from the Tongue River formation water could stimulate the production of methane in coal slurries prepared with coal collected from the Lake De Smet region of the PRB. The Tongue River and Lake De Smet regions lie within the Powder River Basin about 35 miles apart from one another. While the Tongue River area has numerous active wells producing biogenic coal bed methane, generated through metabolic activity of indigenous consortia of MO's, the Lake De Smet region has none. Wells were drilled in the Lake De Smet region but subsequently abandoned because they were unproductive of methane. Figure 2 illustrates the results of these experiments. All incubations were carried out at room temperature, which is similar to *in situ* formation temperatures. Methane production was detected and quantified by gas chromatography. Methane production was detected at relatively low rates in the coal slurries lacking added amendments (unamended controls) also in slurries having an added nutrient solution containing inorganic sources of nitrogen, phosphate, magnesium, calcium, potassium, a variety of metals, and a vitamin mixture. Methane production was not detected in sterilized slurries or in slurries amended with 2-bromoethane sulfonic acid to inhibit methanogenesis. After a short lag period methane production was stimulated significantly with the addition of microorganisms concentrated from the Tongue River formation water. The microbial biomass added to these slurries was equivalent to the number of microorganisms contained within 8.8 ml -of Tongue River formation water. Methanogenesis was also stimulated after a lag period with the addition of Tongue River coal (0.5g) added as a source of inoculum to the Lake De Smet coal slurries. The addition of a termite (*R. flavipes*) hindgut cell suspension as a source of methanogenic consortium to the Lake De Smet coal slurries did not enhance methanogenesis significantly above the unamended coal slurries.

The quantity of cells that could be obtained by concentrating microorganisms from waters in the Powder River Basin (PRB) can be estimated by multiplying the concentration of microorganisms in the Tongue River formation water samples by total PRB CBM water production rate. Based on 44 million barrels/month (6.9 x 10⁹L/month) produced in the PRB in 2004 and 1.2 x 10⁸ cells/L average cell concentration in the Tongue River wells sampled, an estimated 8.4 x 10¹⁷ cells can be recovered from all produced PRB CBM waters each month. The availability of such quantities of demonstrably active methanogenic consortium MO's, using the present invention, has established that it is practical and feasible to enhance methane production from carbonaceous formations, either by seeding a previously unproductive formation, or by stimulating a currently active formation.

Whenever a range is given in the specification, for example, a temperature range, a time range, or a composition or concentration range, all intermediate ranges and subranges, as well as all individual values included in the ranges given are intended to be included in the disclosure.

As used herein, "comprising" is synonymous with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim.

One of ordinary skill in the art will appreciate that starting materials, reagents, solid substrates, synthetic methods, purification methods, and analytical methods other that those specifically exemplified can be employed in the practice of the invention without resort to undue experimentation. All art-known functional equivalents, of any such materials and methods are intended to be included in this invention, to the extent that they are covered by the claims.

A separate but related application, entitled Biogenic Fuel Gas Generation in Geologic Hydrocarbon Deposits has been filed concurrently herewith by inventors Robert Pfeiffer and Roly DeBruyn.

## Claims

1. A method for stimulating methane production from a carbonaceous material comprising the steps of
a) contacting the material with cells of a methanogenic consortium under anaerobic conditions, forming a reaction mixture,
b) maintaining anaerobic conditions for a time sufficient to permit methanogenesis, and
c) collecting methane from anaerobic water or head space of the reaction mixture,
wherein the consortium is a concentrate of microorganisms prepared by the method of
a) extracting anaerobic formation water containing said microorganisms from an underground carbonaceous formation,
b) providing liquid transport means for transporting said water while maintaining an anaerobic state,
c) providing collection means for collecting said microorganisms in an anaerobic state from said water,
d) transporting said water through said collection means whereby said microorganisms are collected from said water, and
e) removing said microorganisms in a concentrated form in said water from the collection means, whereby a concentrate of said microorganisms is prepared.

2. The method of claim 1 wherein the carbonaceous material is present *in situ* in an underground formation.

3. The method of claim 1 wherein the carbonaceous material is extracted from an underground formation.

4. The method of claim 1 wherein the methanogenic consortium is essentially sediment-free.

5. The method of claim 1 wherein the concentrate has at least ten times more microorganisms per unit volume formation water than the formation water of step (a). I

6. The method of claim 1 wherein the anaerobic formation water is obtained from a methane-producing formation.

7. A method of preparing a concentrate of microorganisms comprising a consortium of methanogenic microorganisms, comprising the steps of,
a) extracting anaerobic formation water containing said microorganisms from an underground carbonaceous formation,
b) providing liquid transport means for transporting said water while maintaining an anaerobic state,
c) providing collection means for collecting said microorganisms in an anaerobic state from said water,
d) transporting said water through said collection means, whereby said microorganisms are collected from said water, and
e) removing said microorganisms in a concentrated form in said water from the collection means, whereby a concentrate of said microorganisms is prepared.

8. The method of claim 7 wherein the collection means comprises tangential filtration.

9. The method of claim 7 wherein the collection means comprises centrifugation.

10. The method of claim 7 wherein the concentrate has at least ten times more microorganisms per unit volume than formation water of step (a).

11. The method of claim 7 wherein the concentration contains at least 10⁸ cells/ml.

## Patentansprüche

1. Verfahren zur Stimulation von Methanproduktion aus einem kohlenstoffhältigen Material, das folgende Schritte umfasst:
a) Kontaktieren des Materials mit Zellen eines methanogenen Konsortiums unter anaeroben Bedingungen zur Bildung eines Reaktionsgemischs,
b) Aufrecherhalten der anaeroben Bedingungen ausreichend lange, um eine Methanogenese zu ermöglichen, und
c) Gewinnen von Methan aus anaerobem Wasser oder Kopfraum des Reaktionsgemischs,
wobei das Konsortium ein Konzentrat von Mikroorganismen ist, das durch das folgende Verfahren hergestellt ist:
a) Extrahieren von anaerobem Formationswasser, das die Mikroorganismen enthält, aus einer unterirdischen kohlenstoffhältigen Formation,
b) Bereitstellen eines flüssigen Transportmittels zum Transportieren des Wassers unter Beibehaltung des anaeroben Zustands,
c) Bereitstellen eines Gewinnungsmittels zum Gewinnen der Mikroorganismen in anaerobem Zustand aus dem Wasser,
d) Transportieren des Wassers durch das Gewinnungsmittel, wodurch die Mikroorganismen aus dem Wasser gewonnen werden, und
e) Entfernen der Mikroorganismen in konzentrierter Form im Wasser aus dem Gewinnungsmittel, wodurch ein Konzentrat der Mikroorganismen hergestellt wird.

2. Verfahren nach Anspruch 1, wobei das kohlenstoffhältige Material in situ in einer unterirdischen Formation vorhanden ist.

3. Verfahren nach Anspruch 1, wobei das kohlenstoffhältige Material aus einer unterirdischen Formation extrahiert ist.

4. Verfahren nach Anspruch 1, wobei das methanogene Konsortium im Wesentlichen sedimentfrei ist.

5. Verfahren nach Anspruch 1, wobei das Konzentrat zumindest zehnmal mehr Mikroorganismen pro Einheitsvolumen Formationswasser aufweist als das Formationswasser aus Schritt (a).

6. Verfahren nach Anspruch 1, wobei das anaerobe Formationswasser aus einer methanproduzierenden Formation erhalten wird.

7. Verfahren zur Herstellung eines Konzentrats von Mikroorganismen, das ein Konsortium von methanogenen Mikroorganismen umfasst, folgende Schritte umfassend:
a) Extrahieren von anaerobem Formationswasser, das die Mikroorganismen enthält, aus einer unterirdischen kohlenstoffhältigen Formation,
b) Bereitstellen eines flüssigen Transportmittels zum Transportieren des Wassers unter Beibehaltung des anaeroben Zustands,
c) Bereitstellen eines Gewinnungsmittels zum Gewinnen der Mikroorganismen in anaerobem Zustand aus dem Wasser,
d) Transportieren des Wassers durch das Gewinnungsmittel, wodurch die Mikroorganismen aus dem Wasser gewonnen werden, und
e) Entfernen der Mikroorganismen in konzentrierter Form im Wasser aus dem Gewinnungsmittel, wodurch ein Konzentrat der Mikroorganismen hergestellt wird.

8. Verfahren nach Anspruch 7, wobei das Gewinnungsmittel Tangentialfiltration umfasst.

9. Verfahren nach Anspruch 7, wobei das Gewinnungsmittel Zentrifugation umfasst.

10. Verfahren nach Anspruch 7, wobei das Konzentrat zumindest zehnmal mehr Mikroorganismen pro Einheitsvolumen umfasst als Formationswasser aus Schritt (a).

11. Verfahren nach Anspruch 7, wobei das Konzentrat zumindest 10⁸ Zellen/ml umfasst.

## Revendications

1. Procédé pour stimuler la production de méthane à partir d'un matériau carboné, comprenant les étapes consistant à
a) mettre le matériau en contact avec des cellules d'un consortium méthanogène dans des conditions anaérobies, en formant un mélange réactionnel,
b) maintenir des conditions anaérobies pendant un temps suffisant pour permettre une méthanogenèse, et
c) collecter le méthane à partir de l'eau anaérobie ou d'un espace de tête du mélange réactionnel,
dans lequel le consortium est un concentré de micro-organismes préparé par un procédé consistant à
a) extraire de l'eau de formation anaérobie contenant lesdits micro-organismes à partir d'une formation carbonée souterraine,
b) disposer de moyens de transport de liquide pour transporter ladite eau tout en maintenant un état anaérobie,
c) disposer de moyens de collecte pour collecter lesdits micro-organismes dans un état anaérobie à partir de ladite eau,
d) transporter ladite eau à travers lesdits moyens de collecte de façon que lesdits micro-organismes soient collectés à partir de ladite eau, et
e) retirer lesdits micro-organismes sous une forme concentrée dans l'eau à partir des moyens de collecte, de façon qu'un concentré desdits micro-organismes soit préparé.

2. Procédé selon la revendication 1, dans lequel le matériau carboné est présent in situ dans une formation souterraine.

3. Procédé selon la revendication 1, dans lequel le matériau carboné est extrait à partir d'une formation souterraine.

4. Procédé selon la revendication 1, dans lequel le consortium méthanogène est essentiellement exempt de sédiments.

5. Procédé selon la revendication 1, dans lequel le concentré a au moins dix fois plus de micro-organismes par volume unitaire d'eau de formation que l'eau de formation de l'étape (a).

6. Procédé selon la revendication 1, dans lequel l'eau de formation anaérobie est obtenue à partir d'une formation produisant du méthane.

7. Procédé pour préparer un concentré de micro-organismes comprenant un consortium de micro-organismes méthanogènes, comprenant les étapes consistant à
a) extraire de l'eau de formation anaérobie contenant lesdits micro-organismes à partir d'une formation carbonée souterraine,
b) disposer de moyens de transport de liquide pour transporter ladite eau tout en maintenant un état anaérobie,
c) disposer de moyens de collecte pour collecter lesdits micro-organismes dans un état anaérobie à partir de ladite eau,
d) transporter ladite eau à travers lesdits moyens de collecte de façon que lesdits micro-organismes soient collectés à partir de ladite eau, et
e) retirer lesdits micro-organismes sous une forme concentrée dans l'eau à partir des moyens de collecte, de façon qu'un concentré desdits micro-organismes soit préparé.

8. Procédé selon la revendication 7, dans lequel les moyens de collecte comprennent une filtration tangentielle.

9. Procédé selon la revendication 7, dans lequel les moyens de collecte comprennent une centrifugation.

10. Procédé selon la revendication 7, dans lequel le concentré a au moins dix fois plus de micro-organismes par volume unitaire d'eau de formation que l'eau de formation de l'étape (a).

11. Procédé selon la revendication 7, dans lequel la concentration contient au moins 10⁸ cellules/ml.
